# EUROPEAN PATENT SPECIFICATION

(11) **EP 3 986 546 B1**
(45) Date of publication and mention of the grant of the patent: **18.03.2026**
(21) Application number: 20732615.8
(22) Date of filing: 17.06.2020
(51) Int. Cl.: A61N 5/10, A61B 5/055, A61G 13/12

(54) **CONFIGURABLE RADIOTHERAPY COUCH TOP FOR MAGNETIC RESONANCE RADIOTHERAPY SIMULATION**
KONFIGURIERBARE STRAHLENTHERAPIELIEGEFLÄCHE ZUR SIMULATION EINER MAGNETRESONANZSTRAHLENTHERAPIE
DESSUS DE COUCHETTE DE RADIOTHÉRAPIE CONFIGURABLE POUR SIMULATION DE RADIOTHÉRAPIE À RÉSONANCE MAGNÉTIQUE

(30) Priority: 24.06.2019 EP 19181941
(43) Date of publication of application: 27.04.2022
(73) Proprietor: Koninklijke Philips N.V., 5656 AG Eindhoven (NL)
(72) Inventor: HONKANEN, Antti, Juhani, 5656 AE Eindhoven (NL); DUUS, Ari, 5656 AE Eindhoven (NL); NOUSIAINEN, Jere, Matti, 5656 AE Eindhoven (NL)
(74) Representative: Philips Intellectual Property & Standards
(86) International application number: PCT/EP2020/066810
(87) International publication number: WO 2020/260101

(56) References cited:
- EP-A2- 0 995 397
- WO-A1-2012/080948
- WO-A1-2013/057609
- WO-A1-2016/139352
- WO-A1-2018/029368
- WO-A2-2008/104522
- WO-A2-2013/001399
- WO-A2-2013/046097
- US-A1- 2015 290 066

## Description

### FIELD OF THE INVENTION

The invention relates to Magnetic Resonance Imaging, in particular to magnetic resonance radiotherapy simulation.

### BACKGROUND OF THE INVENTION

Radiotherapy couch tops are flat surfaces used to support subjects in radiotherapy devices. Radiotherapy couch tops typically have systems for mounting restraints and/or fixtures for positioning subjects in a repeatable manner. This allows a subject to be consistently positioned for multiple radiotherapy sessions. Typically, an indexing system which uses rows of mounting points or railings are used to affix the restraints and/or fixtures.

Radiotherapy couch tops may also be mounted to subject supports for magnetic resonance imaging system. This enables the subject to be imaged in a magnetic resonance imaging system using the same subject position that will be later used for a radiotherapy treatment session. Images collected during a magnetic resonance imaging session may therefore be used for planning a subsequent radiotherapy session. The use of a radiotherapy couch top to position a subject during magnetic resonance imaging is referred to a radiotherapy simulation.

WO2013/046097 A2 describes that when imaging a patient using a magnetic resonance (MR) imager, it is desirable to locate a radio frequency (RF) head coil at a position lower than the flat table top patient surface in order to optimize the geometry of the patient anatomy and the RF coil. A flat table top is provided for an MR patient support table that includes a pocket or recessed portion that accepts an RF head coil, hereby optimizing imaging geometry. When the RF coil is not mated to the recessed portion of the table a filler insert is mated therewith.

WO2012/080948 A1 discloses a radiation therapy planning and follow-up system including an MR scanner with a first bore which defines an MR imaging region and a functional scanner, e.g. a nuclear imaging scanner, or a CT scanner with a second bore which defines a nuclear or CT imaging region. The first and second bores have a diameter of at least 70cm, and preferably 80-85 cm. A radiation therapy type couch moves linearly through the MR imaging region along an MR longitudinal axis and the nuclear or CT imaging region along a nuclear or CT longitudinal axis which is aligned with the MR longitudinal axis. The couch positions a subject sequentially in the MR and nuclear or CT imaging regions. A fusion processor combines an image representation generated from data collection in the MR imaging region and an image representation generated from data collection in the nuclear or CT imaging region into a composite image representation and a planning processor generates a radiation therapy treatment plan according to the composite image.

WO2016/139352 A1 describes a patient table comprising: a curved base plate, a support face arranged over the curved base plate's concave side, a planar table top removable placed over the support face surface and the planar table top having a flat support surface opposite form the support face; the planar table top being contiguous to the support face. In particular in the patient table assembly with longitudinal sides (a) longitudinal groove(s) are provided along one or both the longitudinal sides in the flat support surface and at the longitudinal groove(s) indentations, in particular notches, are provided in the flat support surface and transverse to the groove(s).

### SUMMARY OF THE INVENTION

The invention provides for a medical system and a method. The invention is defined by the independent claims. Advantageous embodiments are defined in the dependent claims.

In clinical practice, magnetic resonance imaging systems may be used for both radiotherapy simulation and conventional magnetic resonance imaging procedures. A disadvantage to using a radiotherapy couch top in a magnetic resonance imaging system is that they are flat and relatively uncomfortable. It is therefore undesirable to use a radiotherapy couch top in a magnetic resonance imaging system unless radiotherapy simulation is being performed. To use a magnetic resonance imaging system for both radiotherapy simulation and conventional magnetic resonance imaging, the radiotherapy couch top may be repeatedly installed and uninstalled to change the configuration of the magnetic resonance imaging system. Embodiments of the invention may provide for a means of changing the configuration of a radiotherapy couch top without the need to uninstall it.

This may be achieved by having a subject support with a radiotherapy couch top that has a head support region with a depression or receptacle. The magnetic resonance imaging system may then have a flat head support plate and a magnetic resonance imaging head coil that both fit into the depression. The flat head support plate is installed for radiotherapy simulation and provides the flat surface in the head support region. Installation of the magnetic resonance imaging head coil puts the magnetic resonance imaging system into a configuration useful for conventional clinical magnetic resonance imaging.

In one aspect, the invention provides for a medical system that comprises a magnetic resonance imaging system configured for acquiring magnetic resonance imaging data from a subject within an imaging zone. The medical system further comprises a subject support configured for supporting at least a portion of the subject within the imaging zone. The subject support comprises a radiotherapy couch top configured for receiving the subject. The radiotherapy couch top comprises a flat surface configured for supporting the subject. The radiotherapy couch top further comprises a head support region configured for receiving a head of the subject. The head region comprises a depression. The depression may alternatively be referred to as a magnetic resonance imaging antenna receptacle. The magnetic resonance imaging antenna receptacle may for example be configured for receiving a head coil. The head region is configured for receiving a flat head support plate. The medical system further comprises the flat head support plate. The flat head support plate is configured to form part of the flat surface when installed in the head region.

The subject support has the depression, and the flat head support plate when installed covers the depression such that this region then becomes an extension of the flat surface. This may be beneficial because in radiotherapy couch tops the subject is mounted on a flat surface for radiotherapy. The installation of the flat head support plate enables the subject support to simulate a radiotherapy couch top that would be used for a radiotherapy system. This may be beneficial because it may enable the use of a magnetic resonance imaging system for a radiotherapy simulation as well as for conventional magnetic resonance imaging protocols without the need to uninstall the radiotherapy couch top.

In another embodiment, the depression has a curved profile.

The flat head support plate comprises radiotherapy mask attachment fixtures. A radiotherapy mask may for example also be referred to as an S-type mask. Radiotherapy masks may be designed to immobilize a head region of a subject. The presence of the radiotherapy mask attachment fixtures on the flat head support plate enables the improved simulation of radiotherapy procedures using the magnetic resonance imaging system.

In another embodiment, the depression is configured for mounting a magnetic resonance imaging head coil. The depression may be a receptacle that is configured for mounting or receiving the magnetic resonance imaging head coil.

In another embodiment, the medical system comprises the magnetic resonance imaging head coil. In this embodiment the medical system comprises both the flat head support plate and the magnetic resonance imaging head coil. The flat head support plate or the magnetic resonance imaging head coil may either be installed into the radiotherapy couch top. When the flat head support plate is installed then the magnetic resonance imaging system is configured for simulating radiotherapy procedures. When the magnetic resonance imaging head coil is installed then the magnetic resonance imaging system may be used for conventional magnetic resonance imaging protocols. This may be beneficial because it may enable the medical system to be used for a larger variety of magnetic resonance imaging protocols.

In another embodiment the flat head support plate is installed in the head region.

The medical system further comprises a memory for storing machine-executable instructions and pulse sequence commands. The pulse sequence commands are configured to control the magnetic resonance imaging system to acquire the magnetic resonance imaging data. The medical system further comprises a processor for controlling the medical system. Execution of the machine-executable instructions causes the processor to control the magnetic resonance imaging system with pulse sequence commands to acquire the magnetic resonance imaging data. Execution of the machine-executable instructions further causes the processor to reconstruct at least one magnetic resonance image using the magnetic resonance imaging data.

This may be beneficial because it may be useful for performing the radiotherapy simulation. The references to the pulse sequence commands as used herein may refer to more than one set of pulse sequence commands. For example, the memory of the magnetic resonance imaging system may comprise a library or database of various pulse sequence commands and may be selected for the appropriate procedure.

Execution of the machine-executable instructions further causes the processor to generate radiation therapy planning data using the at least one magnetic resonance image. When the subject is constrained or attached to the radiotherapy couch top the subject will be in the same position as when the subject is placed into a radiotherapy system. The images that are then acquired with the subject on the radiotherapy couch top may therefore be useful for generating radiation therapy planning data. This for example may indicate the location of target regions within the subject as well as sensitive organs which are intended to be avoided during radiotherapy.

In another embodiment, generation of the radiation therapy planning data comprises applying an organ contouring algorithm to the at least one magnetic resonance image to generate an organ segmentation. This may for example be done using an anatomical atlas or a deformable shape model (or another organ contouring algorithm). This may be beneficial in providing improved radiation therapy planning data.

In another embodiment, the pulse sequence commands are configured for at least partially acquiring the magnetic resonance imaging data according to a diffusion weighted magnetic resonance imaging protocol. Generation of the radiation therapy planning data comprises reconstructing the at least one diffusion weighted magnetic resonance image for radiotherapy dose sculpting. The use of the diffusion weighted magnetic resonance imaging data may be useful because the diffusion weighted images may illustrate the location of tumors and other lesions. The at least one diffusion weighted magnetic resonance image may then be useful for performing radiotherapy dose sculpting which is to control the amount of radiation into different regions of an organ or other volume of a subject. This may provide for improved subject health as well as the effectiveness of any radiotherapy.

In another embodiment, execution of the machine-executable instructions further causes the processor to receive a radiotherapy treatment plan. A radiotherapy treatment plan as used herein encompasses instructions or data which may be used to detail what portions of a subject to irradiate and which portions to minimize radiation exposure to. This may also include a prescription or instructions for a particular dose for certain target regions of the subject.

Execution of the machine-executable instructions further causes the processor to generate radiotherapy treatment system control commands using the radiotherapy treatment plan, the radiotherapy planning data, and a radiotherapy system model. For example, the radiotherapy planning data may be registered to the radiotherapy treatment plan. The radiotherapy system model may model the behavior and control functionality of a radiotherapy system. The radiotherapy system control commands are then commands which may be used for controlling the radiotherapy system which is modeled by the radiotherapy system model. This embodiment may be beneficial because it may provide for an improved means of controlling a radiotherapy system.

In another embodiment, execution of the machine-executable instructions further causes the processor to reconstruct at least one pseudo-CT image using the magnetic resonance imaging data. A pseudo-CT image as used herein is a simulation of a CT image that is reconstructed using one or more magnetic resonance images. For example, segmentations of a magnetic resonance image may be used to identify different tissue type regions within a subject. A knowledge of the X-ray absorption of various types of tissue may then be used to reconstruct the pseudo-CT image. This may be beneficial because CT images are used by many radiotherapy planning systems to control radiotherapy systems.

The pulse sequence commands are further configured to acquire the magnetic resonance imaging data according to any one of the following: an ultra-short echo time magnetic resonance imaging protocol, according to a T1 weighted magnetic resonance imaging protocol, according to a T2 weighted magnetic resonance imaging protocol, and combinations thereof. The ultra-short echo time magnetic resonance imaging protocol may be useful in imaging cortical bone. This may provide for an improved pseudo-CT image. The use of the T2 weighted and T1 weighted magnetic resonance images may be used for effectively identifying various tissue types or tissue regions. They may also provide for improved pseudo-CT images.

The use of T2 weighted as used herein may also encompass T2* weighting. If T1 and/or T2 weighted images are used for the reconstruction of the pseudo-CT images and an anatomical atlas or other model such as a deformable shape model may be used to accurately determine the composition of different regions within the subject using the magnetic resonance images.

In another embodiment, the medical system further comprises a memory for storing machine-executable instructions and pulse sequence commands. Again, the pulse sequence commands may refer to a selection of pulse sequence commands that can be retrieved according to the exact protocol or requirements for a particular subject. The pulse sequence commands are configured to control the magnetic resonance imaging system to acquire the magnetic resonance imaging data. The medical system further comprises a processor for controlling the medical system. Execution of the machine-executable instructions further causes the processor to control the magnetic resonance imaging system with the pulse sequence commands to acquire the magnetic resonance imaging data. The pulse sequence commands are configured to cause the magnetic resonance imaging system to acquire the magnetic resonance imaging data using the magnetic resonance imaging head coil.

Execution of the machine-executable instructions further causes the processor to reconstruct at least one magnetic resonance image using the magnetic resonance imaging data. In this embodiment the medical system is used with the magnetic resonance imaging system behaving as a conventional magnetic resonance imaging system. In this case there is no restriction on the type of magnetic resonance imaging protocol that can be performed. The intent is that in this embodiment the magnetic resonance imaging system functions as a general magnetic resonance imaging system used for a diagnosis as would be found in a clinic or hospital.

In another embodiment, the radiotherapy couch top is rigid. The use of a radiotherapy couch top may be beneficial because the radiotherapy couch top is always in a fixed position with relation to the various mounts and extra points on it. When a subject is mounted to the radiotherapy couch top that is rigid the subject will always be in the same position even if the subject is repositioned multiple times.

In another aspect, the invention provides for a method of operating a medical system. The medical system comprises a magnetic resonance imaging system configured for acquiring magnetic resonance imaging data from a subject within an imaging zone. The medical system further comprises a subject support configured for supporting at least a portion of the subject within the imaging zone. The subject support comprises a radiotherapy couch top configured for receiving the subject. The radiotherapy couch top comprises a flat surface.

The radiotherapy couch top further comprises a head support region configured for receiving a head of a subject. The head region comprises a depression. The head region is configured for receiving a flat head support plate. The method comprises installing the flat head support plate. The flat head support plate is configured to form a part of a flat surface when installed in the head region. The method further comprises controlling the magnetic resonance imaging system with the pulse sequence commands to acquire the magnetic resonance imaging data. This magnetic resonance imaging data may for example also be called first magnetic resonance imaging data.

In another embodiment, the depression is configured for receiving a magnetic resonance imaging head coil. The method further comprises removing the flat head support plate support. The method further comprises installing the magnetic resonance imaging head coil in the depression. The method further comprises controlling the magnetic resonance imaging system with the pulse sequence commands to acquire the magnetic resonance imaging data using the head coil. The pulse sequence commands used to acquire the magnetic resonance imaging data when the magnetic resonance imaging head coil is installed may also be referred to as second pulse sequence commands and the magnetic resonance imaging data may be referred to as second magnetic resonance imaging data.

It is understood that one or more of the aforementioned embodiments of the invention may be combined as long as the combined embodiments are not mutually exclusive.

As will be appreciated by one skilled in the art, aspects of the present invention may be embodied as an apparatus or a method. Accordingly, aspects of the present invention may take the form of an entirely hardware embodiment, or an embodiment combining software (including firmware, resident software, micro-code, etc.) and hardware aspects that may all generally be referred to herein as a "circuit," "module" or "system." Furthermore, aspects of the present invention may use a computer program product embodied in one or more computer-readable medium(s) having computer-executable code embodied thereon.

Any combination of one or more computer-readable medium(s) may be utilized. The computer-readable medium may be a computer-readable signal medium or a computer-readable storage medium. A 'computer-readable storage medium' as used herein encompasses any tangible storage medium which may store instructions which are executable by a processor of a computing device. The computer-readable storage medium may be referred to as a computer-readable non-transitory storage medium. The computer-readable storage medium may also be referred to as a tangible computer-readable medium. In some embodiments, a computer-readable storage medium may also be able to store data which is able to be accessed by the processor of the computing device. Examples of computer-readable storage media include, but are not limited to: a floppy disk, a magnetic hard disk drive, a solid state hard disk, flash memory, a USB thumb drive, Random Access Memory (RAM), Read Only Memory (ROM), an optical disk, a magneto-optical disk, and the register file of the processor. Examples of optical disks include Compact Disks (CD) and Digital Versatile Disks (DVD), for example CD-ROM, CD-RW, CD-R, DVD-ROM, DVD-RW, or DVD-R disks. The term computer-readable-storage medium also refers to various types of recording media capable of being accessed by the computer device via a network or communication link. For example, data may be retrieved over a modem, over the internet, or over a local area network. Computer-executable code embodied on a computer-readable medium may be transmitted using any appropriate medium, including but not limited to wireless, wire line, optical fiber cable, RF, etc., or any suitable combination of the foregoing.

A computer-readable signal medium may include a propagated data signal with computer-executable code embodied therein, for example, in baseband or as part of a carrier wave. Such a propagated signal may take any of a variety of forms, including, but not limited to, electro-magnetic, optical, or any suitable combination thereof. A computer-readable signal medium may be any computer-readable medium that is not a computer-readable storage medium and that can communicate, propagate, or transport a program for use by or in connection with an instruction execution system, apparatus, or device.

'Computer memory' or 'memory' is an example of a computer-readable storage medium. Computer memory is any memory which is directly accessible to a processor. 'Computer storage' or 'storage' is a further example of a computer-readable storage medium. Computer storage is any non-volatile computer-readable storage medium. **In** some embodiments computer storage may also be computer memory or vice versa.

A 'processor' as used herein encompasses an electronic component which is able to execute a program or machine-executable instruction or computer-executable code. References to the computing device comprising "a processor" should be interpreted as possibly containing more than one processor or processing core. The processor may for instance be a multi-core processor. A processor may also refer to a collection of processors within a single computer system or distributed amongst multiple computer systems. The term computing device should also be interpreted to possibly refer to a collection or network of computing devices each comprising a processor or processors. The computer-executable code may be executed by multiple processors that may be within the same computing device or which may even be distributed across multiple computing devices.

Computer-executable code may comprise machine-executable instructions or a program which causes a processor to perform an aspect of the present invention. Computer-executable code for carrying out operations for aspects of the present invention may be written in any combination of one or more programming languages, including an object-oriented programming language such as Java, Smalltalk, C++ or the like and conventional procedural programming languages, such as the "C" programming language or similar programming languages and compiled into machine-executable instructions. **In** some instances, the computer-executable code may be in the form of a high-level language or in a pre-compiled form and be used in conjunction with an interpreter which generates the machine-executable instructions on the fly.

The computer-executable code may execute entirely on the user's computer, partly on the user's computer, as a stand-alone software package, partly on the user's computer and partly on a remote computer or entirely on the remote computer or server. In the latter scenario, the remote computer may be connected to the user's computer through any type of network, including a local area network (LAN) or a wide area network (WAN), or the connection may be made to an external computer (for example, through the Internet using an Internet Service Provider).

Aspects of the present invention are described with reference to flowchart illustrations and/or block diagrams of methods, apparatus (systems) and computer program products according to embodiments of the invention. It is understood that each block or a portion of the blocks of the flowchart, illustrations, and/or block diagrams, can be implemented by computer program instructions in form of computer-executable code when applicable. It is further understood that, when not mutually exclusive, combinations of blocks in different flowcharts, illustrations, and/or block diagrams may be combined. These computer program instructions may be provided to a processor of a general-purpose computer, special purpose computer, or other programmable data processing apparatus to produce a machine, such that the instructions, which execute via the processor of the computer or other programmable data processing apparatus, create means for implementing the functions/acts specified in the flowchart and/or block diagram block or blocks.

These computer program instructions may also be stored in a computer-readable medium that can direct a computer, other programmable data processing apparatus, or other devices to function in a particular manner, such that the instructions stored in the computer-readable medium produce an article of manufacture including instructions which implement the function/act specified in the flowchart and/or block diagram block or blocks.

The computer program instructions may also be loaded onto a computer, other programmable data processing apparatus, or other devices to cause a series of operational steps to be performed on the computer, other programmable apparatus or other devices to produce a computer implemented process such that the instructions which execute on the computer or other programmable apparatus provide processes for implementing the functions/acts specified in the flowchart and/or block diagram block or blocks.

A `user interface' as used herein is an interface which allows a user or operator to interact with a computer or computer system. A 'user interface' may also be referred to as a 'human interface device.' A user interface may provide information or data to the operator and/or receive information or data from the operator. A user interface may enable input from an operator to be received by the computer and may provide output to the user from the computer. In other words, the user interface may allow an operator to control or manipulate a computer, and the interface may allow the computer to indicate the effects of the operator's control or manipulation. The display of data or information on a display or a graphical user interface is an example of providing information to an operator. The receiving of data through a keyboard, mouse, trackball, touchpad, pointing stick, graphics tablet, joystick, gamepad, webcam, headset, pedals, wired glove, remote control, and accelerometer are all examples of user interface components which enable the receiving of information or data from an operator.

A 'hardware interface' as used herein encompasses an interface which enables the processor of a computer system to interact with and/or control an external computing device and/or apparatus. A hardware interface may allow a processor to send control signals or instructions to an external computing device and/or apparatus. A hardware interface may also enable a processor to exchange data with an external computing device and/or apparatus. Examples of a hardware interface include, but are not limited to: a universal serial bus, IEEE 1394 port, parallel port, IEEE 1284 port, serial port, RS-232 port, IEEE-488 port, Bluetooth connection, Wireless local area network connection, TCP/IP connection, Ethernet connection, control voltage interface, MIDI interface, analog input interface, and digital input interface.

A 'display' or 'display device' as used herein encompasses an output device or a user interface adapted for displaying images or data. A display may output visual, audio, and or tactile data. Examples of a display include, but are not limited to: a computer monitor, a television screen, a touch screen, tactile electronic display, Braille screen,
Cathode ray tube (CRT), Storage tube, Bi-stable display, Electronic paper, Vector display, Flat panel display, Vacuum fluorescent display (VF), Light-emitting diode (LED) displays, Electroluminescent display (ELD), Plasma display panels (PDP), Liquid crystal display (LCD), Organic light-emitting diode displays (OLED), a projector, and Head-mounted display.

Magnetic resonance imaging data or magnetic resonance data is defined herein as being the recorded measurements of radio frequency signals emitted by atomic spins using the antenna of a Magnetic resonance apparatus during a magnetic resonance imaging scan. Magnetic resonance data is an example of medical image data. A Magnetic Resonance Imaging (MRI) image or MR image is defined herein as being the reconstructed two- or three-dimensional visualization of anatomic data contained within the magnetic resonance imaging data. This visualization can be performed using a computer.

### BRIEF DESCRIPTION OF THE DRAWINGS

In the following preferred embodiments of the invention will be described, by way of example only, and with reference to the drawings in which:
Fig. 1 illustrates an example of a radiotherapy couch top mounted to a subject support of a magnetic resonance imaging system;
Fig. 2 illustrates an example of a flat head support plate;
Fig. 3 shows a further view of the radiotherapy couch top and subject support of Fig. 1;
Fig. 4 illustrates an example of a medical system;
Fig. 5 shows a flow chart which illustrates a method of operating the medical system of Fig. 4;
Fig. 6 shows a further view of the medical system of Fig. 4,
Fig. 7 shows a flow chart which illustrates a method of operating the medical system as configured in Fig. 6; and
Fig. 8 shows a flow chart which illustrates a further method of operating the medical system illustrated in Figs. 4 and 6.

### DETAILED DESCRIPTION OF THE EMBODIMENTS

Like numbered elements in these figures are either equivalent elements or perform the same function. Elements which have been discussed previously will not necessarily be discussed in later figures if the function is equivalent.

Medical imaging may be used as a basis for treatment planning in external beam radiotherapy. In radiotherapy devices (such as Linear accelerators), the patient table (radiotherapy couch top) is flat and equipped with indexing for patient positioning devices while in diagnostic imaging MR scanners the patient tables typically are not flat (e.g curved form is often used). When using MR scanner for radiotherapy simulation (imaging) the curved table top is replaced by the flat table top.

The receive coils and other accessories of the MR scanner are typically designed for the curved form table. While the receive coils and accessories are designed for other table form (curved) they cannot be optimally used with the flat table.

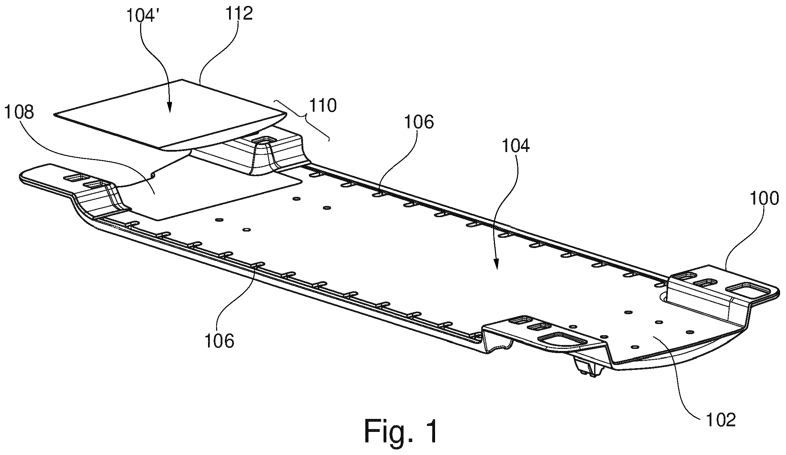

Fig. 1 depicts an example of a subject support 100 for a magnetic resonance imaging system. The subject support 100 has a radiotherapy couch top 102 that has been installed in the top. The radiotherapy couch top 102 is an insert for the subject support 100 that is fixed into place and replicates a radiotherapy couch top as would be used for a radiotherapy system. The radiotherapy couch top 102 has a flat surface 104 and an indexing system 106. In this example the indexing system is a collection of two rows of parallel holes or mounts to which fixtures can be attached. The use of such holes or fixtures is typical. In other examples the index system 106 may be two railings or rails to which fixtures may be attached that on either side of the flat surface 104.

It can be seen in this example that there is a depression 108 or coil receptacle in a head support region 110. The drawing depicts a flat head support plate 112 that may be installed into the subject support 100 so that the depression 108 is covered. The flat head support plate 112 also has a flat surface 104' that forms part of the flat surface 104 when it is installed.

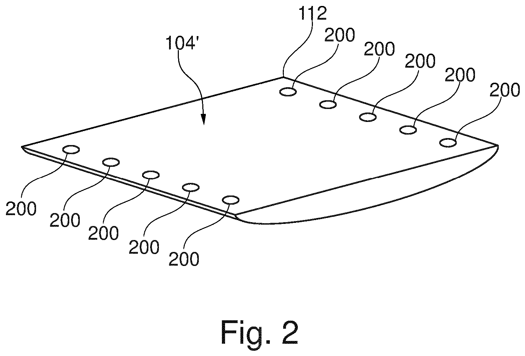

Fig. 2 illustrates an alternative flat head support plate 112. The flat head support plate 112 in Fig. 2 may be used to replace the flat head support plate 112 depicted in Fig. 1. The flat head support plate 112 in Fig. 2 is similar to the flat head support plate 112 in Fig. 1 with the exception of a number of radiotherapy mask attachment fixtures 200. In this example the radiotherapy mask attachment fixtures 200 are two rows of poles which may be threaded or configured for receiving a mount. For example, a radiotherapy mask or S-type mask may be fixed to the flat head support plate 112.

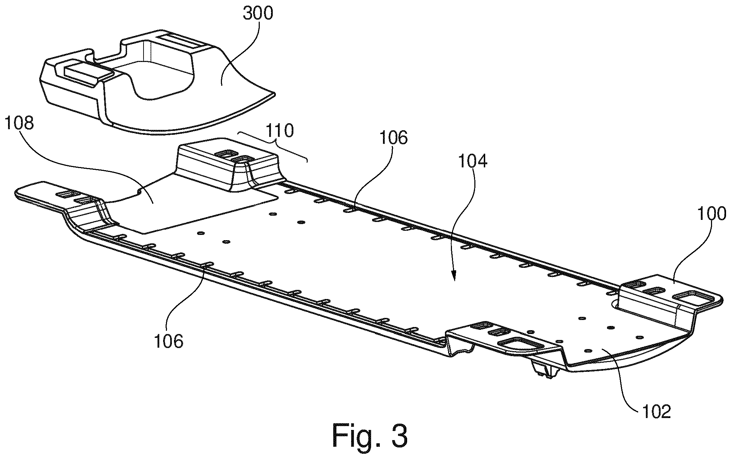

Fig. 3 shows a further view of the magnetic resonance imaging support 100 depicted in Fig. 1. In the diagram in Fig. 3 the flat head support plate 112 is not shown. Instead there is a magnetic resonance imaging head coil 300 which can be installed into the depression 108 or coil receptacle. From Figs. 1 and 3 it is very clear that the subject support 100 can be very quickly and easily switched between two configurations. The configuration for Fig. 1 may be used for radiation therapy simulation. The configuration depicted in Fig. 3 may be used for conventional magnetic resonance imaging protocols. This may be achieved without the need to remove or replace the radiotherapy couch top 102.

In this example, the flat table top (flat surface 10) dedicated for Radio Therapy (RT) simulation imaging is equipped with the (curved) features (depression 108) which allow placing the diagnostic RF coils 300 or other accessories in optimal position. In case the RF coil is not needed for the application, the coil can be replaced by a filler piece.

The flat head support plate 112 functions as a filler piece. In case the RF coil is not needed for the application, the coil can be replaced by a filler piece to form a continuous flat surface that may be used for RT simulation.

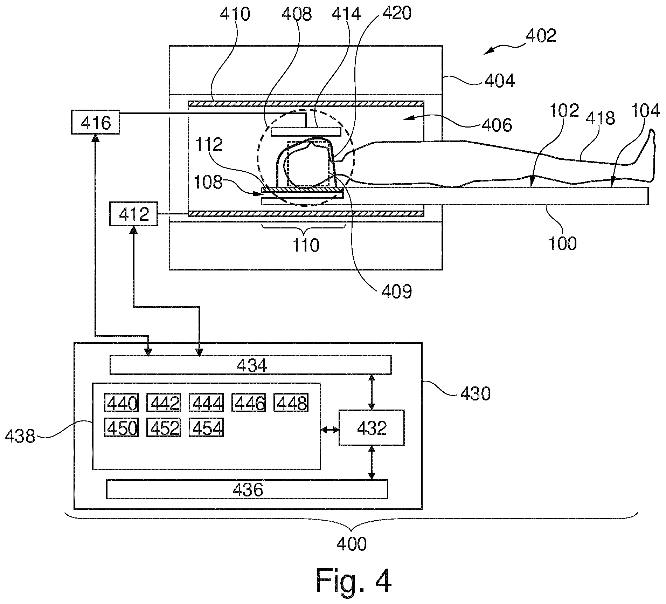

Fig. 4 illustrates an example of a medical system 400. The medical system 400 comprises a magnetic resonance imaging system 402, the subject support 100 depicted in Figs. 1 and 3, and a computer system 430.

The magnetic resonance imaging system 402 comprises a magnet 404. The magnet 404 is a superconducting cylindrical type magnet with a bore 406 through it. The use of different types of magnets is also possible; for instance, it is also possible to use both a split cylindrical magnet and a so-called open magnet. A split cylindrical magnet is similar to a standard cylindrical magnet, except that the cryostat has been split into two sections to allow access to the iso-plane of the magnet, such magnets may for instance be used in conjunction with charged particle beam therapy. An open magnet has two magnet sections, one above the other with a space in-between that is large enough to receive a subject: the arrangement of the two sections area similar to that of a Helmholtz coil. Open magnets are popular, because the subject is less confined. Inside the cryostat of the cylindrical magnet there is a collection of superconducting coils.

Within the bore 406 of the cylindrical magnet 404 there is an imaging zone 408 where the magnetic field is strong and uniform enough to perform magnetic resonance imaging. A field of view 409 is shown within the imaging zone 408. The magnetic resonance data that is acquired acquired for the field of view 409. A subject 418 is shown as being supported by the subject support 100 such that a head region of the subject 418 is within the imaging zone 408 and the field of view 409.

Within the bore 406 of the magnet there is also a set of magnetic field gradient coils 410 which is used for acquisition of preliminary magnetic resonance data to spatially encode magnetic spins within the imaging zone 408 of the magnet 404. The magnetic field gradient coils 410 connected to a magnetic field gradient coil power supply 412. The magnetic field gradient coils 410 are intended to be representative. Typically, magnetic field gradient coils 410 contain three separate sets of coils for spatially encoding in three orthogonal spatial directions. A magnetic field gradient power supply supplies current to the magnetic field gradient coils. The current supplied to the magnetic field gradient coils 410 is controlled as a function of time and may be ramped or pulsed.

Adjacent to the imaging zone 408 is a radio-frequency coil 414 for manipulating the orientations of magnetic spins within the imaging zone 408 and for receiving radio transmissions from spins also within the imaging zone 408. The radio frequency antenna may contain multiple coil elements. The radio frequency antenna may also be referred to as a channel or antenna. The radio-frequency coil 414 is connected to a radio frequency transceiver 416. The radio-frequency coil 414 and radio frequency transceiver 416 may be replaced by separate transmit and receive coils and a separate transmitter and receiver. It is understood that the radio-frequency coil 414 and the radio frequency transceiver 416 are representative. The radio-frequency coil 414 is intended to also represent a dedicated transmit antenna and a dedicated receive antenna. Likewise, the transceiver 416 may also represent a separate transmitter and receivers. The radio-frequency coil 414 may also have multiple receive/transmit elements and the radio frequency transceiver 416 may have multiple receive/transmit channels. For example, if a parallel imaging technique such as SENSE is performed, the radio-frequency could 414 will have multiple coil elements.

The subject support 100 is shown with the flat head support plate 112 installed. The subject 418 is reposing on the flat surface 104. A radiotherapy mask 420 is shown as restraining the head of the subject 418. The subject's 418 head is within the field of view 409. With the flat head support plate 112 installed the flat surface 104' as depicted in Figs. 1 and 2 form part of the flat surface 104.

The radio-frequency transmitter 416 and the magnetic field gradient coil power supply 412 are shown as being connected to a hardware interface 434 of computer system 430. The computer system 430 further comprises a processor 432 that is shown as being connected with the hardware interface 434, a user interface 436, and a memory 438. The processor 432 is intended to represent one or more processors that may be distributed in one or more computing systems. The memory 438 is also intended to represent any sort of memory or storage which is accessible to the processor 432.

The memory 438 is shown as containing machine-executable instructions 440. The machine-executable instructions 440 enable the processor 432 to control the operation and function of the medical system 400 as well as to perform basic data and image processing procedures. The memory 438 is further shown as containing pulse sequence commands 442. The pulse sequence commands 442 may represent one or more sets of pulse sequence commands that may be used to control the magnetic resonance imaging system to acquire magnetic resonance imaging data. The pulse sequence commands may also be data which may be converted into such commands.

The memory 438 is further shown as containing magnetic resonance imaging data 444 that has been acquired by controlling the magnetic resonance imaging system 402 with the pulse sequence commands 442. The memory 438 is further shown as containing a magnetic resonance image 446 that has been reconstructed from the magnetic resonance imaging data 444. The magnetic resonance image 446 may represent two or three-dimensional image data and may represent multiple images.

The memory 438 is further shown as containing radiation therapy planning data 448. The radiation therapy planning data 448 is data which may be used for radiotherapy simulation. For example, the radiation therapy planning data 448 may be magnetic resonance images 446 which have been segmented and used to identify various regions of tissue for eradiation or to reduce the minimal amount of radiation exposure to.

The memory 438 is shown as further containing optional radiotherapy treatment plan 450. This for example may incorporate anatomical data and/or previously specified data which represents a region of the subject 418 to irradiate as well as possibly regions which are intended to not be irradiate or to reduce the amount of exposed radiation to. The memory 438 is shown as optionally containing a radiotherapy system model 452. The radiotherapy system model 452 may for example be used for simulating the behavior of a particular radiotherapy system.

The memory 438 is further shown as optionally containing radiotherapy system control commands 454 which were constructed using the radiation therapy planning data 448, the optional radiotherapy treatment plan 450, and the optional radiotherapy system model 452. The radiotherapy system control commands 454 may be actual commands for controlling the radiotherapy system modeled by the radiotherapy system model 452 to perform an eradiation of the subject 418. The features 450, 452, and 454 may be in a different distributed computer system. They may for example be incorporated into a radiotherapy system.

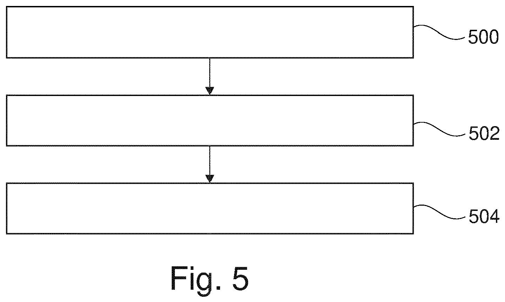

Fig. 5 illustrates a method of operating the medical system 400 of Fig. 4. It is noted that the method in Fig. 5 is performed with the flat head support plate 112 installed. First in step 500 the magnetic resonance imaging system 402 is controlled with the pulse sequence commands 442 to acquire the magnetic resonance imaging data 444. Next in step 502 the at least one magnetic resonance image 446 is reconstructed from the magnetic resonance imaging data 444. Next in step 504 the radiation therapy planning data 448 is reconstructed from the at least one magnetic resonance image 446. This for example may be achieved by an implementation of an organ contouring or segmentation algorithm.

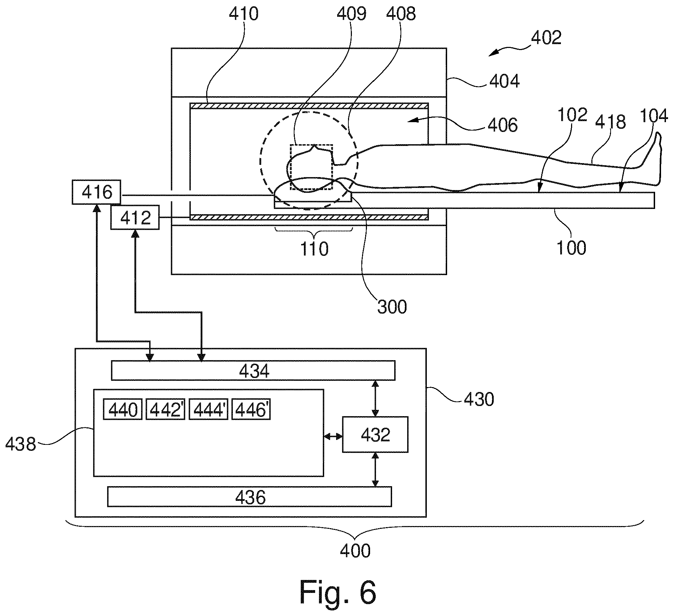

Fig. 6 shows a further view of the medical system 400 of Fig. 4. **In** this example the flat head support plate 112 has been removed and instead the magnetic resonance imaging head coil 300 has been installed into the subject support 100. **In** the configuration illustrated in Fig. 6 the magnetic resonance imaging system 402 may be used for conventional clinical magnetic resonance imaging.

The memory 438 is again shown as containing the machine-executable instructions 440. The memory 438 is shown as containing the pulse sequence commands 442'. These may be different pulse sequence commands than were illustrated in Fig. 4. The pulse sequence commands 442' may also be referred to as second pulse sequence commands. The memory 438 is further shown as containing magnetic resonance imaging data 444' that has been acquired by controlling the magnetic resonance imaging system 402 with the pulse sequence commands 442'. The magnetic resonance imaging data 444' may also be referred to as second magnetic resonance imaging data. The memory 438 is shown as containing at least one magnetic resonance image 446' that has been reconstructed from the magnetic resonance imaging data 444'. The magnetic resonance images 446' may also be referred to as second magnetic resonance images.

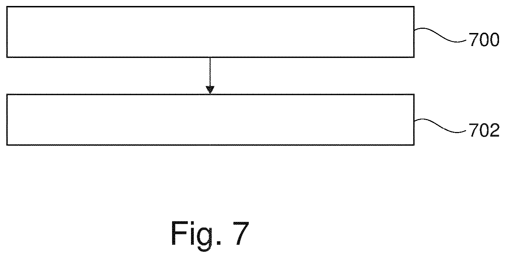

Fig. 7 illustrates a method of operating the medical system 400 as is depicted in Fig. 6. The method in Fig. 7 may be executed when the magnetic resonance imaging head coil 300 is installed into the subject support 100. First in step 700 the magnetic resonance imaging system 402 is controlled with the pulse sequence commands 442' to acquire the magnetic resonance imaging data 444'. Then, in step 702, the at least one magnetic resonance image 446' is reconstructed using the magnetic resonance imaging data 444'.

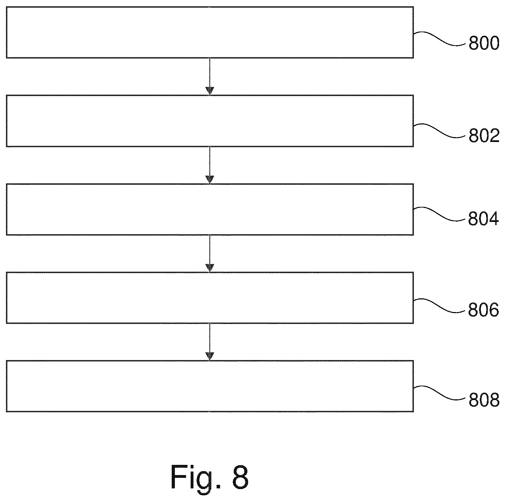

Fig. 8 shows a flowchart which illustrates a method of operating and configuring the medical system 400 that is depicted in Figs. 4 and 6. First in step 800 the flat head support plate 112 is installed into the subject support. Next in step 802, the magnetic resonance imaging system is controlled with the pulse sequence commands 442 to acquire the magnetic resonance imaging data 444. The pulse sequence commands 442 may be referred to as first pulse sequence commands and the magnetic resonance imaging data 444 may also be referred to as first magnetic resonance imaging data.

Next in step 804, the flat head support plate 112 is removed. Then in step 806 the magnetic resonance imaging head coil 300 is installed into the depression 108 or receptacle. Finally, in step 808, the magnetic resonance imaging system 402 is controlled with the pulse sequence commands 442' to acquire the magnetic resonance imaging data 444'. Again, the pulse sequence commands 442' may be referred to as second pulse sequence commands and the magnetic resonance imaging data 444' may be referred to as second magnetic resonance imaging data.

While the invention has been illustrated and described in detail in the drawings and foregoing description, such illustration and description are to be considered illustrative or exemplary and not restrictive; the invention is not limited to the disclosed embodiments.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measures cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

### LIST OF REFERENCE NUMERALS

- 100: subject support
- 102: radiotheraphy couch top
- 104: flat surface
- 104': flat surface
- 106: index system
- 108: depression or coil recepticle
- 110: head support region
- 112: flat head support plate
- 200: radiotherapy mask attachment fixtures
- 300: magnetic resonance imaging head coil
- 400: medical system
- 402: magnetic resonance imaging system
- 404: magnet
- 406: bore of magnet
- 408: imaging zone
- 409: field of view
- 410: magnetic field gradient coils
- 412: magnetic field gradient coil power supply
- 414: radio-frequency coil
- 416: transceiver
- 418: subject
- 420: radiotherapy mask
- 430: computer
- 432: processor
- 434: hardware interface
- 436: user interface
- 438: memory
- 440: machine-executable instructions
- 442: pulse sequence commands
- 442': pulse sequence commands
- 444: magnetic resonance imaging data
- 444': magnetic resonance imaging data
- 446: magnetic resonance image
- 446': magnetic resonance image
- 448: radiation therapy planning data
- 450: radiotheraphy treatment plan
- 452: radiotherapy system model
- 454: radiotherapy system control commands
- 502: control the magnetic resonance imaging system with the pulse sequence commands to acquire the magnetic resonance imaging data
- 504: reconstruct at least one magnetic resonance image using the magnetic resonance imaging data
- 506: generate radiation therapy planning data using the at least one magnetic resonance image
- 700: control the magnetic resonance imaging system with the pulse sequence commands to acquire the magnetic resonance imaging data
- 702: reconstruct at least one magnetic resonance image using the magnetic resonance imaging data
- 800: installing the flat head support plate
- 802: controlling the magnetic resonance imaging system with pulse sequence commands to acquire the magnetic resonance imaging data
- 804: removing the flat head support plate
- 806: installing the magnetic resonance imaging head coil in the depression
- 808: controlling the magnetic resonance imaging system with pulse sequence commands to acquire the magnetic resonance imaging data using the head coil

## Claims

1. A medical system (400) comprising:
a magnetic resonance imaging system (402) configured for acquiring magnetic resonance imaging data (444, 444') from a subject (418) within an imaging zone (408);
a subject support (100) configured for supporting at least a portion of the subject within the imaging zone, wherein the subject support comprises a radiotherapy couch top (102) configured for receiving the subject, wherein the radiotherapy couch top comprises a flat surface (104) configured for supporting the subject, wherein the radiotherapy couch top further comprises a head support region (110) configured for receiving a head of the subject, wherein the head region comprises a depression (108), and wherein the head region is configured for receiving a flat head support plate (112); and
the flat head support plate (112), wherein the flat head support plate is configured to form part of the flat surface (104') when installed in the head region, wherein the flat head support plate comprises radiotherapy mask attachment fixtures (200); wherein the flat head support plate is installed in the head region,
a memory (438) for storing machine-executable instructions (440) and pulse sequence commands (442), wherein the pulse sequence commands are configured to control the magnetic resonance imaging system to acquire the magnetic resonance imaging data (444), and
a processor (432) for controlling the medical system, wherein execution of the machine-executable instructions causes the processor to:
- control (502) the magnetic resonance imaging system with the pulse sequence commands (442) to acquire the magnetic resonance imaging data (444);
- reconstruct (504) at least one magnetic resonance image (446) using the magnetic resonance imaging data; and
- generate (506) radiation therapy planning data (448) using the at least one magnetic resonance image.

2. The medical system of claim 1, wherein the depression is configured for mounting a magnetic resonance imaging head coil (300).

3. The medical system of claim 2, wherein the medical system comprises the magnetic resonance imaging head coil.

4. The medical system of any one of claims 1 through 3, wherein generation of the radiotherapy planning data comprises applying an organ contouring algorithm to the at least one magnetic resonance image to generate an organ segmentation.

5. The medical system of any one of the preceding claims, wherein the pulse sequence commands are configured for at least partially acquiring the magnetic resonance imaging data according to a diffusion weighted magnetic resonance imaging protocol, wherein generation of the radiotherapy planning data comprises reconstructing at least one diffusion weighted magnetic resonance image for radiotherapy dose sculpting.

6. The medical system of any one of the preceding claims, wherein execution of the machine-executable instructions further causes the processor to:
- receive a radiotherapy treatment plan (450); and
- generate radiotherapy treatment system control commands using the radiotherapy treatment plan, the radiotherapy planning data, and a radiotherapy system model (452).

7. The medical system of any one of the preceding claims, wherein execution of the machine-executable instructions further causes the processor to reconstruct at least one pseudo CT images using the magnetic resonance imaging data, wherein the pulse sequence commands are configured to acquire the magnetic resonance imaging data according to any one of the following: according to an ultrashort echo time magnetic resonance imaging protocol, according to a T1 weighted magnetic resonance imaging protocol, according to a T2 weighted magnetic resonance imaging protocol, and combinations thereof.

8. The medical system of any one of the preceding claims, wherein the radiotherapy couch top is rigid.

9. A method of operating a medical system (400), wherein the medical system comprises a magnetic resonance imaging system (402) configured for acquiring magnetic resonance imaging data (444, 444') from a subject (418) within an imaging zone (408), wherein the medical system further comprises a subject support (100) configured for supporting at least a portion of the subject within the imaging zone, wherein the subject support comprises a radiotherapy couch top (102) configured for receiving the subject, wherein the radiotherapy couch top comprises a flat surface (104), wherein the radiotherapy couch top further comprises a head support region (110) configured for receiving a head of the subject, wherein the head region comprises a depression (108), wherein the head region is configured for receiving a flat head support plate (112), wherein the flat head support plate comprises radiotherapy mask attachment fixtures (200), wherein the method comprises:
- installing (800) the flat head support plate, wherein the flat head support plate is configured to form part of the flat surface (104') when installed in the head region;
- controlling (802) the magnetic resonance imaging system with pulse sequence commands (442) to acquire the magnetic resonance imaging data (444); and
- generate (506) radiation therapy planning data (448) using the at least one magnetic resonance image.

10. The method of claim 9, wherein the depression is configured for receiving a magnetic resonance imaging head coil (300), wherein the method further comprises;
- removing (804) the flat head support plate;
- installing (806) the magnetic resonance imaging head coil in the depression; and
- controlling (808) the magnetic resonance imaging system with pulse sequence commands (442') to acquire the magnetic resonance imaging data (444') using the head coil.

## Patentansprüche

1. Medizinisches System (400), umfassend:
ein Magnetresonanztomographiesystem (402), das zum Erfassen von Magnetresonanztomographiedaten (444, 444') von einem Patienten (418) innerhalb einer Bildgebungszone (408) konfiguriert ist;
eine Patientenlagerungsvorrichtung (100), die dazu konfiguriert ist, mindestens einen Abschnitt des Patienten innerhalb der Bildgebungszone zu lagern, wobei die Patientenlagerungsvorrichtung eine Auflage für die Strahlentherapie-Liege (102) umfasst, die zum Aufnehmen des Patienten konfiguriert ist, wobei die Auflage für die Strahlentherapie-Liege eine ebene Fläche (104) umfasst, die zum Lagern des Patienten konfiguriert ist, wobei die Auflage für die Strahlentherapie-Liege weiter einen Kopflagerungsbereich (110) umfasst, der zum Aufnehmen eines Kopfes des Patienten konfiguriert ist, wobei der Kopfbereich eine Vertiefung (108) umfasst und wobei der Kopfbereich zum Aufnehmen einer flachen Kopflagerungsplatte (112) konfiguriert ist; und
die flache Kopflagerungsplatte (112), wobei die flache Kopflagerungsplatte dazu konfiguriert ist, beim Einbauen im Kopfbereich einen Teil der flachen Fläche (104') zu bilden, wobei die flache Kopflagerungsplatte Befestigungselemente (200) für die Strahlentherapiemaske umfasst; wobei die flache Kopflagerungsplatte im Kopfbereich eingebaut ist,
einen Speicher (438) zum Speichern von maschinenausführbaren Anweisungen (440) und Pulssequenzbefehlen (442), wobei die Pulssequenzbefehle dazu konfiguriert sind, das Magnetresonanztomographiesystem zum Erfassen der Magnetresonanztomographiedaten (444) zu steuern, und
einen Prozessor (432) zum Steuern des medizinischen Systems, wobei Ausführung der maschinenausführbaren Anweisungen den Prozessor dazu veranlasst:
- das Magnetresonanztomographiesystem mit den Pulssequenzbefehlen (442) zum Erfassen der Magnetresonanztomographiedaten (444) zu steuern (502);
- mindestens ein Magnetresonanzbild (446) anhand der Magnetresonanztomographiedaten zu rekonstruieren (504); und
- Strahlentherapieplanungsdaten (448) unter Verwendung des mindestens eines Magnetresonanzbildes zu generieren (506).

2. Medizinisches System nach Anspruch 1, wobei die Vertiefung zur Montage einer Magnetresonanztomographie-Kopfspule (300) konfiguriert ist.

3. Medizinisches System nach Anspruch 2, wobei das medizinische System die Magnetresonanztomographie-Kopfspule umfasst.

4. Medizinisches System nach einem der Ansprüche 1 bis 3, wobei das Generieren der Strahlentherapieplanungsdaten das Anwenden eines Organkonturierungsalgorithmus auf das mindestens eine Magnetresonanzbild zum Generieren einer Organsegmentierung umfasst.

5. Medizinisches System nach einem der vorstehenden Ansprüche, wobei die Pulssequenzbefehle dazu konfiguriert sind, die Magnetresonanztomographiedaten zumindest teilweise gemäß einem diffusionsgewichteten MagnetresonanztomographieProtokoll zu erfassen, wobei das Generieren der Strahlentherapieplanungsdaten das Rekonstruieren mindestens eines diffusionsgewichteten Magnetresonanzbildes zur Strahlentherapiedosisformung umfasst.

6. Medizinisches System nach einem der vorstehenden Ansprüche, wobei das Ausführen
der maschinenausführbaren Anweisungen den Prozessor weiter dazu veranlassen:
- einen Strahlentherapiebehandlungsplan zu empfangen (450); und
- Steuerbefehle für das Strahlentherapiebehandlungssystem unter Verwendung des Strahlentherapie-Behandlungsplans, der Strahlentherapieblanungsdaten und eines Strahlentherapiesystemmodells zu generieren (452).

7. Medizinisches System nach einem der vorstehenden Ansprüche, wobei die Ausführung der maschinenausführbaren Anweisungen den Prozessor weiter dazu veranlasst, mindestens ein Pseudo-CT-Bild unter Verwendung der Magnetresonanztomographiedaten zu rekonstruieren, wobei die Pulssequenzbefehle dazu konfiguriert sind, Magnetresonanztomographiedaten nach einem der folgenden Protokolle zu erfassen: nach einem Magnetresonanztomographieprotokoll mit ultrakurzer Echozeit, nach einem T1-gewichteten Magnetresonanztomographieprotokoll, nach einem T2-gewichteten Magnetresonanztomographieprotokoll oder Kombinationen davon.

8. Medizinisches System nach einem der vorstehenden Ansprüche, wobei die Auflage für die Strahlentherapie-Liege starr ist.

9. Verfahren zum Betrieb eines medizinischen Systems (400), wobei das medizinische System ein Magnetresonanztomographiesystem (402) umfasst, das dazu konfiguriert ist, Magnetresonanztomographiedaten (444, 444') von einem Patienten (418) innerhalb einer Bildgebungszone (408) zu erfassen, wobei das medizinische System weiter eine Patientenlagerungsvorrichtung (100) umfasst, die dazu konfiguriert ist, mindestens einen Abschnitt des Patienten innerhalb der Bildgebungszone zu lagern, wobei die Patientenlagerungsvorrichtung eine Auflage für die Strahlentherapie-Liege (102) umfasst, die zum Aufnehmen des Patienten konfiguriert ist, wobei die Auflage für die Strahlentherapie-Liege eine flache Fläche (104) umfasst, wobei die Auflage für die Strahlentherapie-Liege weiter einen Kopflagerungsbereich (110) umfasst, der zum Aufnehmen eines Kopfes des Patienten konfiguriert ist, wobei der Kopfbereich eine Vertiefung (108) umfasst, wobei der Kopfbereich zum Aufnehmen einer flachen Kopflagerungsplatte (112) konfiguriert ist, wobei die flache Kopflagerungsplatte Befestigungselemente (200) für die Bestrahlungsmaske aufweist, wobei das Verfahren Folgendes umfasst:
- Einbauen (800) der flachen Kopflagerungsplatte, wobei die Flache Kopflagerungsplatte dazu konfiguriert ist, beim Einbauen im Kopfbereich einen Teil der flachen Fläche (104') zu bilden;
- Steuern (802) des Magnetresonanztomographiesystems mit Pulssequenzbefehlen (442) zum Erfassen der Magnetresonanztomographiedaten (444); und
- Generieren (506) von Strahlentherapieplanungsdaten (448) unter Verwendung des mindestens eines Magnetresonanzbildes.

10. Verfahren nach Anspruch 9, wobei die Vertiefung zum Aufnehmen einer Magnetresonanztomographie-Kopfspule (300) konfiguriert ist, wobei das Verfahren weiter Folgendes umfasst:
- Entfernen (804) der flachen Kopflagerungsplatte;
- Einbauen (806) der Magnetresonanztomographie-Kopfspule in der Vertiefung; und
- Steuern (808) des Magnetresonanztomographiesystems mit Pulssequenzbefehlen (442') zum Erfassen der Magnetresonanztomographiedaten (444') unter Verwendung der Kopfspule.

## Revendications

1. Système médical (400), comprenant :
un système d'imagerie par résonance magnétique (402) configuré pour acquérir des données d'imagerie par résonance magnétique (444, 444') d'un sujet (418) dans une zone d'imagerie (408) ;
un support de sujet (100) configuré pour supporter au moins une partie du sujet dans la zone d'imagerie, dans lequel le support de sujet comprend un plateau de table de radiothérapie (102) configuré pour recevoir le sujet, dans lequel le plateau de table de radiothérapie comprend une surface plane (104) configurée pour supporter le sujet, dans lequel le plateau de table de radiothérapie comprend en outre une région de support de tête (110) configurée pour recevoir la tête du sujet, dans lequel la région de tête comprend une dépression (108), et dans lequel la région de tête est configurée pour recevoir une plaque de support de tête plane (112) ; et
la plaque de support de tête plane (112), dans lequel la plaque de support de tête plane est configurée pour faire partie de la surface plane (104') lorsqu'elle est installée dans la région de tête, dans lequel la plaque de support de tête plane comprend des fixations pour masque de radiothérapie (200) ; dans lequel la plaque de support de tête plane est installée dans la région de tête,
une mémoire (438) pour stocker des instructions exécutables par machine (440) et des commandes de séquence d'impulsions (442), dans lequel les commandes de séquence d'impulsions sont configurées pour commander le système d'imagerie par résonance magnétique afin d'acquérir les données d'imagerie par résonance magnétique (444), et
un processeur (432) destiné à commander le système médical, dans lequel l'exécution des instructions exécutables par machine amène le processeur à :
- commander (502) le système d'imagerie par résonance magnétique avec les commandes de séquence d'impulsions (442) pour acquérir les données d'imagerie par résonance magnétique (444) ;
- reconstruire (504) au moins une image par résonance magnétique (446) à l'aide des données d'imagerie par résonance magnétique ; et
- générer (506) des données de planification de radiothérapie (448) à l'aide de la au moins une image de résonance magnétique.

2. Système médical selon la revendication 1, dans lequel la dépression est configurée pour monter une bobine de tête d'imagerie par résonance magnétique (300).

3. Système médical selon la revendication 2, dans lequel le système médical comprend la bobine de tête d'imagerie par résonance magnétique.

4. Système médical selon l'une quelconque des revendications 1 à 3, dans lequel la génération des données de planification de radiothérapie comprend l'application d'un algorithme de contourage d'organe à la au moins une image de résonance magnétique pour générer une segmentation d'organe.

5. Système médical selon l'une quelconque des revendications précédentes, dans lequel les commandes de séquence d'impulsions sont configurées pour acquérir au moins partiellement les données d'imagerie par résonance magnétique selon un protocole d'imagerie par résonance magnétique pondérée en diffusion, dans lequel la génération des données de planification de radiothérapie comprend la reconstruction d'au moins une image par résonance magnétique pondérée en diffusion pour la balistique de radiothérapie.

6. Système médical selon l'une quelconque des revendications précédentes, dans lequel l'exécution des instructions exécutables par machine amène en outre le processeur à :
- recevoir un plan de traitement de radiothérapie (450) ; et
- générer des commandes de régulation de système de traitement de radiothérapie à l'aide du plan de traitement de radiothérapie, des données de planification de radiothérapie et d'un modèle de système de radiothérapie (452).

7. Système médical selon l'une quelconque des revendications précédentes, dans lequel l'exécution des instructions exécutables par machine amène en outre le processeur à reconstruire au moins une pseudo image CT à l'aide des données d'imagerie par résonance magnétique, dans lequel les commandes de séquence d'impulsions sont configurées pour acquérir les données d'imagerie par résonance magnétique selon l'un quelconque des protocoles suivants : selon un protocole d'imagerie par résonance magnétique à temps d'écho ultracourt, selon un protocole d'imagerie par résonance magnétique pondéré T1, selon un protocole d'imagerie par résonance magnétique pondéré T2, et des combinaisons de ceux-ci.

8. Système médical selon l'une quelconque des revendications précédentes, dans lequel le plateau de table de radiothérapie est rigide.

9. Procédé de fonctionnement d'un système médical (400), dans lequel le système médical comprend un système d'imagerie par résonance magnétique (402) configuré pour acquérir des données d'imagerie par résonance magnétique (444, 444') d'un sujet (418) à l'intérieur d'une zone d'imagerie (408), dans lequel le système médical comprend en outre un support de sujet (100) configuré pour supporter au moins une partie du sujet dans la zone d'imagerie, dans lequel le support de sujet comprend un plateau de table de radiothérapie (102) configuré pour recevoir le sujet, dans lequel le plateau de table de radiothérapie comprend une surface plane (104), dans lequel le plateau de table de radiothérapie comprend en outre une région de support de tête (110) configurée pour recevoir la tête du sujet, dans lequel la région de tête comprend une dépression (108), dans lequel la région de tête est configurée pour recevoir une plaque de support de tête plane (112), dans lequel la plaque de support de tête plane comprend des fixations pour masque de radiothérapie (200), dans lequel le procédé comprend les étapes consistant à :
- installer (800) la plaque de support de tête plane, dans lequel la plaque de support de tête plane est configurée pour faire partie de la surface plane (104') lorsqu'elle est installée dans la région de tête ;
- commander (802) le système d'imagerie par résonance magnétique avec des commandes de séquence d'impulsions (442) pour acquérir les données d'imagerie par résonance magnétique (444) ; et
- générer (506) des données de planification de radiothérapie (448) à l'aide de la au moins une image de résonance magnétique.

10. Procédé selon la revendication 9, dans lequel la dépression est configurée pour recevoir une bobine de tête d'imagerie par résonance magnétique (300), dans lequel le procédé comprend en outre les étapes consistant à :
- retirer (804) la plaque de support de tête plane ;
- installer (806) la bobine de tête d'imagerie par résonance magnétique dans la dépression ; et
- commander (808) le système d'imagerie par résonance magnétique avec des commandes de séquence d'impulsions (442) pour acquérir les données d'imagerie par résonance magnétique (444) à l'aide de la bobine de tête.
